# EUROPEAN PATENT SPECIFICATION

(11) **EP 2 996 608 B1**
(45) Date of publication and mention of the grant of the patent: **08.12.2021**
(21) Application number: 14721108.0
(22) Date of filing: 14.03.2014
(51) Int. Cl.: A61B 34/20, H05K 1/18, A61B 5/06, H05K 1/16

(54) **NAVIGATED SURGICAL INSTRUMENT**
NAVIGIERTES CHIRURGISCHES INSTRUMENT
INSTRUMENT CHIRURGICAL À NAVIGATION

(30) Priority: 15.03.2013 US 201361790479 P; 13.03.2014 US 201414209696
(43) Date of publication of application: 23.03.2016
(73) Proprietor: Medtronic Navigation, Inc., Louisville, CO 80027 (US)
(72) Inventor: STRUPECK, Frank, Louisville, Colorado 80027 (US); JAIN, Abhishek, Centennial, Colorado 80016 (US); BURG, Bruce M., Louisville, Colorado 80027 (US); JACOBSEN, Brad, Erie, Colorado 80516 (US)
(74) Representative: Dehns
(86) International application number: PCT/US2014/028100
(87) International publication number: WO 2014/143919

(56) References cited:
- WO-A1-97/29684
- WO-A1-2010/124285
- US-A1- 2006 206 170
- US-A1- 2013 066 194
- None

## Description

### FIELD

The present disclosure relates generally to a navigated surgical instrument and, more particularly, to a navigated surgical instrument having a plurality of navigation coils defined on a flexible circuit.

### BACKGROUND

The statements in this section merely provide background information related to the present disclosure and may not constitute prior art.

In surgical navigation systems, instruments are provided with tracking devices. Sometimes, however, such tracking devices can be difficult to manipulate or cumbersome to the instrument. In other instances, the tracking devices can be positioned in a handle or proximal region of the instrument such that if the distal tip moves or is moved relative to the handle, the distal tip can no longer be accurately tracked.

In many instances, tracking devices contain tracking coils that must be accurately positioned within the surgical instrument. To reduce induced electrical noise, the surgical instruments often utilize twisted pairs of leads that can be expensive to form and must be accommodated in the construction of the medical device. Moreover, because of their size, the tracking coils, and often the leads, are difficult to electronically couple to the navigation system. Navigated surgical instruments are taught in WO 97/29684 and US 2013/0066194.

WO 97/29684 discloses a probe comprising a body defining a lumen and a plurality of transducer assemblies mounted to said body around said lumen. Each transducer assembly includes one or more transducers such as coils or other transducers. A first transducer assembly includes a first coil such as a helical coil having turns encircling the lumen and having a coil axis extending in a first direction codirectional with said lumen. A second transducer assembly includes a pair of second coils disposed on opposite sides of the lumen. The second coils have axes extending in a second direction transverse to said first direction. The second coils are aligned with one another in the first direction but are offset from one another in a third direction transverse to said first and second directions. The probe further includes a third transducer assembly including a pair of third coils disposed on opposite sides of said lumen, the axes of said third coils extending in said third direction, the axes of said third coils being offset from one another in said second direction.

### SUMMARY

The invention is defined in the appended set of claims.

This section provides a general summary of the disclosure, and is not a comprehensive disclosure of its full scope or all of its features.

In a first form, a tracking device for a medical device navigation system associated with a medical instrument having a shaft is disclosed. The tracking device has an elongated tubular substrate having an exterior surface and a longitudinal axis. The tracking device has a first electrical trace defining a first coil, the first coil configured to interact with the navigation system.

In another form, a surgical instrument is provided and can include an elongated body portion, a tracking device, and a handle portion. The tracking device can be formed on a flexible circuit which defines at least one coil positioned adjacent or near a distal end of the surgical instrument. The tracking device can be adapted to cooperate with a navigation system to track the distal end of the instrument.

In another form, a surgical instrument includes an elongated tubular body portion, and a monolithic tubular flexible circuit portion having a trace defining a navigation coil. The flexible circuit portion can have a proximal end, a distal end, and an inner diameter defining a first internal passage between the proximal and distal ends received on the outer diameter of the body portion.

In another form, the tracking device can be coupled to the body portion adjacent to the distal tip, and can be adapted to cooperate with a navigation system to track the distal tip. A handle portion can be coupled to the proximal end of the body portion. The tracking device can include at least a pair of lead traces defined on the tubular flexible circuit portion and around the body portion at an acute angle relative to a longitudinal axis of the body portion. The tubular flexible circuit portion can further define a plurality of coils formed as traces on the flexible circuit. A flexible outer layer can cover the body portion, the flexible circuit having pair of lead traces and tracking device.

Further areas of applicability will become apparent from the description provided herein. It should be understood that the description and specific examples are intended for purposes of illustration only and are not intended to limit the scope of the present teachings.

### BRIEF DESCRIPTION OF THE DRAWINGS

The present teachings will become more fully understood from the detailed description, the appended claims and the following drawings. The drawings are for illustrative purposes only and are not intended to limit the scope of the present disclosure.
Figure 1 is a perspective view of an exemplary navigation system according to the principles of the present disclosure;
Figure 2 is a top plan view of an exemplary instrument for use with the navigation system according to the principles of the present disclosure;
Figures 3-5 are a perspective view of the exemplary tracking devices according to the principles of the present disclosure;
Figure 6 is a partial cross-sectional view of the flexible circuit portion; and
Figure 7 is an exemplary surgical instrument according to the present teachings.

### DETAILED DESCRIPTION OF THE VARIOUS EMBODIMENTS

The following description is merely exemplary in nature and is in no way intended to limit the present disclosure, its application, or uses. It should also be understood that throughout the drawings, corresponding reference numerals indicate like or corresponding parts and features.

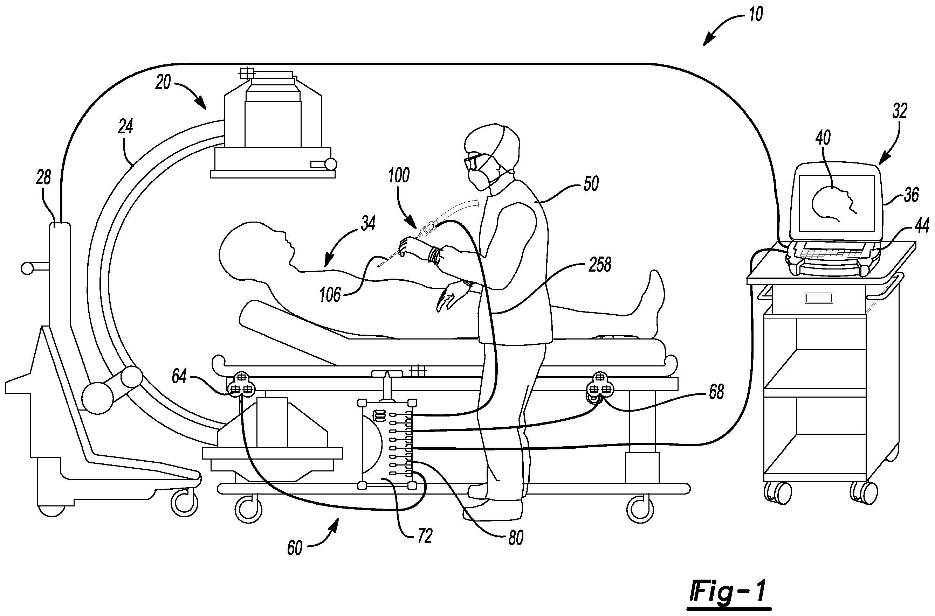

Figure 1 is a diagram schematically illustrating an overview of an image-guided navigation system 10 for use in the non-line-of-site navigating of a surgical instrument 100, such as a navigable malleable suction device or suction instrument, according to various exemplary embodiments of the present disclosure. Exemplary navigation systems include those disclosed in U.S. Patent No. 7,366,562, entitled "Method and Apparatus for Surgical Navigation," issued on April 29, 2008 and U.S. Patent No. 8,320,991, entitled "Portable Electromagnetic Navigation System," issued on November 27, 2012. Commercial navigation systems include the StealthStation^{®} AxiEM^{™} Surgical Navigation System sold by Medtronic Navigation, Inc. having a place of business in Louisville, Colorado, USA. It should be appreciated that while the navigation system 10 and instrument 100 are generally described in connection with an ear, nose and throat (ENT) procedure, navigation system 10 and instrument 100 can be used in various other appropriate procedures.

Generally, the navigation system 10 can be used to track a location of instrument 100, including a distal tip or end thereof, as will be described herein. Navigation system 10 can generally include an optional imaging system 20, such as a fluoroscopic X-ray imaging device configured as a C-arm 24 and an image device controller 28. The C-arm imaging system 20 can be any appropriate imaging system, such as a digital or CCD camera, which are well understood in the art. Image data obtained can be stored in the C-arm controller 28 and sent to a navigation computer and/or processor controller or work station 32 having a display device 36 to display image data 40 and a user interface 44. The work station 32 can also include or be connected to an image processor, navigation processor, and a memory to hold instruction and data. The work station 32 can include an optimization processor that assists in a navigated procedure. It will also be understood that the image data is not necessarily first retained in the controller 28, but may also be directly transmitted to the workstation 32. Moreover, processing for the navigation system and optimization can all be done with a single or multiple processors all of which may or may not be included in the work station 32.

The work station 32 provides facilities for displaying the image data 40 as an image on the display device 36, saving, digitally manipulating, or printing a hard copy image of the received image data. The user interface 44, which may be a keyboard, mouse, touch pen, touch screen or other suitable device, allows a physician or user 50 to provide inputs to control the imaging device 20, via the C-arm controller 28, or adjust the display settings of the display device 36.

With continuing reference to Figure 1, the navigation system 10 can further include a tracking system, such as an electromagnetic (EM) tracking system 60. The discussion of the EM tracking system 60 can be understood to relate to any appropriate tracking system. The EM tracking system 60 can include a localizer, such as a coil array 64 and/or second coil array 68, a coil array controller 72, a navigation probe interface 80, and the trackable instrument 100. Instrument 100 can include an instrument tracking device or devices 106, as will be discussed herein. Briefly, the tracking device 106 can include an electromagnetic coil to sense a field produced by the localizing coil arrays 64, 68 and provide information to the navigation system 10 to determine a location of the tracking device 106. The navigation system 10 can then determine a position of a distal tip of the instrument 100 to allow for navigation relative to the patient 34 and patient space.

The EM tracking system 60 can use the coil arrays 64, 68 to create an electromagnetic field used for navigation. The coil arrays 64, 68 can include a plurality of coils that are each operable to generate distinct electromagnetic fields into the navigation region of the patient 34, which is sometimes referred to as patient space. Optionally, time and frequency division multiplexing can be used to generate distinct electromagnetic fields into the navigation region. Representative electromagnetic systems are set forth in U.S. Patent No. 5,913,820, entitled "Position Location System," issued June 22, 1999 and U.S. Patent No. 5,592,939, entitled "Method and System for Navigating a Catheter Probe," issued January 14, 1997.

The coil arrays 64, 68 can be controlled or driven by the coil array controller 72. The coil array controller 72 can drive each coil in the coil arrays 64, 68 in a time division multiplex, a frequency division multiplex and combinations thereof. In this regard, each coil may be driven separately at a distinct time or all of the coils may be driven simultaneously with each being driven by a different frequency.

Upon driving the coils in the coil arrays 64, 68 with the coil array controller 72, electromagnetic fields are generated within the patient 34 in the area where the medical procedure is being performed, which is again sometimes referred to as patient space. The coil arrays 64, 68 and coil array controller 72 can produce unique field strengths and directions. The electromagnetic fields generated in the patient space induce currents in the tracking device 106 positioned on or in the instrument 100. These induced signals from the tracking device 106 can be delivered to the navigation probe interface 80 and subsequently forwarded to the coil array controller 72. The navigation probe interface 80 can also include amplifiers, filters and buffers to directly interface with the tracking device 106 in the instrument 100. Alternatively, the tracking device 106, or any other appropriate portion, may employ a traceless communications channel, such as that disclosed in U.S. Patent No. 6,474,341, entitled "Surgical Communication Power System," issued November 5, 2002, as opposed to being coupled directly to the navigation probe interface 80.

The tracking system 60, if it is using an electromagnetic tracking assembly, essentially works by positioning the coil arrays 64, 68 adjacent to the patient 34 to generate a magnetic field, which can be low energy, and generally referred to as a navigation field. Because every point in the navigation field or patient space is associated with unique field strength and directions, the electromagnetic tracking system 60 can determine the position of the instrument 100 by measuring the field strength at the tracking device 106 location. The coil array controller 72 can receive the induced signals from the tracking device 106 and transmit information regarding a location, where location information can include both x, y, and z position and roll, pitch, and yaw orientation information, of the tracking device 106 associated with the tracked instrument 100. Accordingly, six degrees of freedom (6 DOF) information can be determined with the navigation system 10.

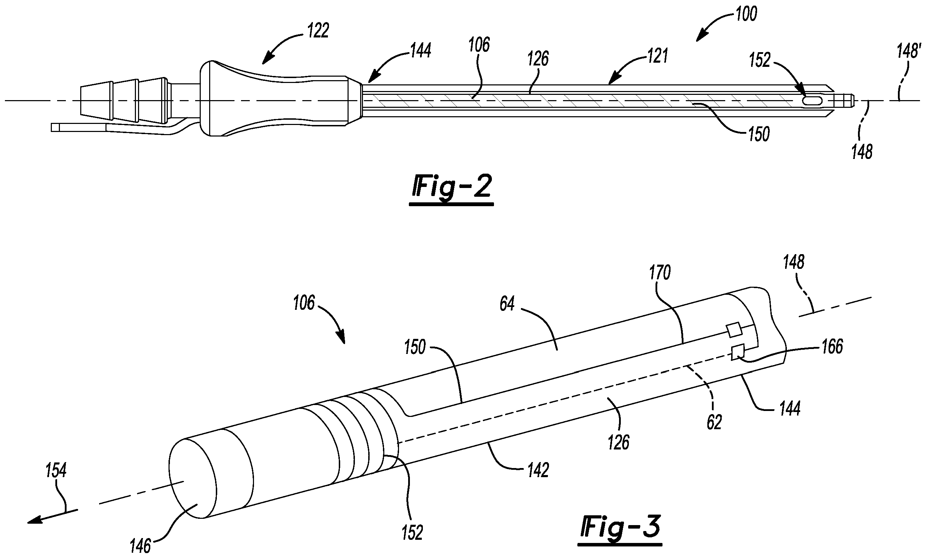

Referring now to Figures 2-7, the navigated surgical instrument 100 will be described in greater detail. While the surgical instrument 100 is shown in one exemplary configuration as a suction device, those skilled in the art will realize the instrument 100 can have a solid elongated shaft. In the disclosed configuration, the instrument 100 can be used for suction, including fluid and tissue removal in ENT procedures. It should be appreciated; however, that the instrument 100 can be used in various other surgical procedures as may be desired and can be provided in the form of a malleable or flexible endoscope, a malleable or flexible catheter, and/or a malleable cannula a solid tool or cutting member support. Thus, while the following description continues with reference to a navigated instrument 100, the discussion is also applicable to other surgical instruments and other surgical procedures. For example, it is envisioned that the antenna configuration can be used to determine the location of non-tubular medical instruments by the incorporation of the coils therein or thereon.

Instrument 100 can have an elongated cylindrical portion 121, a handle 122 and a tracking device 106. The instrument can be configured for multiple use or for a single use such that it would be disposed after such use. The tracking device 106 has a tubular body 126 having traces 150 which define one or more tracking coils 152. As described further below, a proximal end 144 of the tubular body 126 can function as an electrical couple to the navigation system 10. The elongated cylindrical portion 121, tracking device 106 and tracking coils 152 can be surrounded by a polymeric outer heat shrink 272 covering the entire assembly.

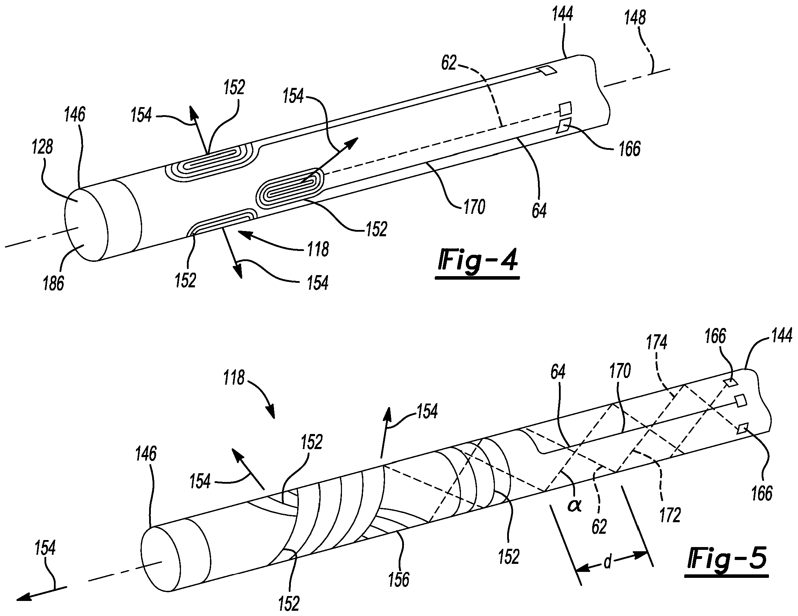

Shown generally in Figures 3-5 is the tracking device 106 according to the present teachings. As shown in Figure 2, the tracking device 106 has a tubular body 126 formed of a tubular substrate 142 with both the proximal end 144 and distal end 146. The tubular body 126 defines a longitudinal axis 148, which can generally align with the longitudinal axis 148' of the medical instrument 100. The conductive trace 150 is circumferentially disposed or "wrapped" around a directional axis 154 associated with the tubular body 126. As shown in Figure 3, the directional axis 154 can be aligned with or parallel to the longitudinal axis 148 defined by the tubular substrate 142 by wrapping the trace-defined coil 152 around an exterior surface of the tubular body 126. Alternatively, as shown in Figures 4 and 5, the directional axis 154 can be an axis that is not aligned and is non-parallel to the longitudinal axis 148 of the tubular body 126. Optionally, trace-defined coil 152 can be formed at an intermediate layer of the flexible circuit.

As shown in Figures 4 and 5, different sets of coils 152 formed using the non-aligned axis 154 can be combined and formed on the substrate 142. In this regard, as shown in Figure 4, the coils 152 can be axially displaced along the length of the substrate 142 from the proximal and distal ends 144, 146. The coils 152 can be circular or elliptical, defining a generally concave shape with respect to the cylindrical instrument. This concave shape can be useful in defining the vector direction. In this regard, rotating a cylindrical coil in a plane perpendicular to the axis 148 can change the direction of the detection axis 154 due to the change in convex shape of the coil 152. Each coil 152 can be disposed about an axis 154 that is non-parallel to the longitudinal axis 148. When the axis 154 is perpendicular to an exterior surface 140 of the tubular substrate 142, the coils 152 form pads that conform in shape to the curvature of the exterior surface 140. Optionally, the center of the various coils can be displaced along the axis, and radially positioned about the axis. In this configuration, the coils 152 can be positioned so that while the coils are radially displaced, the coil centers are axial displaced. Additionally, the trace-defined coils 152 can be overlapped, by placing individual trace-defined coils 152 on different laminar layers of the tubular substrate 142.

As shown in Figure 5, the axis 154 can be generally non-perpendicular to the exterior surface 140 of the substrate 142, thus forming angled coils 152. It is envisioned a tracking coil cluster 156 can be formed of two or three coils 152 whose axis 154 can be different (e.g., orthogonal). The tracking coil cluster 156 is formed of several overlaying layers of traces 150 and insulating substrate, as further described herein. The traces 150 can have lead traces 162, 164 which electrically connect the coils 152 with electrical coupling pads 166 defined on the proximal end 144 of the substrate. The electrical coupling pads 166 allow the tracking device 106 to be electrically coupled to the navigation system 10. Should the traces be formed on a single side of the substrate, no vias will be necessary. Alternatively, the traces can be formed by twisted wires as is known in the art. As shown in the Figures 3-5, these coupling traces 162, 164 can be linear 170, helical 172, or form twisted pairs 174. A further discussion of navigation coils 152 can be found in U.S. Patent Publication No. 2010/0210939, entitled "Method and Apparatus for Surgical Navigation," published August 19, 2010.

The tubular tracking device 106 can be formed using several methods. In this regard, a tubular body 126 can be a printed circuit board formed first on a planar flexible circuit 232 which contains the coils 152 and conductive traces 150, defined on one or more layers of the flexible circuit 232. This tubular body 126 can then be wound to define a hollow tubular shape. This shape can be held in place using an adhesive along the gap interface 186 (see Figure 4). Alternatively, the tubular body 126 can be formed on a tubular polymer substrate 128 (see Figures 3 and 5). The coils 152 and conductive traces 150 can be disposed onto the exterior surface 140 of the tubular polymer substrate 128. As described in detail below, covering insulative layers 240 can then be disposed over the coils 152 and conductive traces 150. It is envisioned the traces 150, which can be placed on an exterior surface of the substrate or an inter-laminar layer of the tubular body 146, can be coupled to the coils 152 using vias. Alternatively, the tubular body can be formed by helically winding the flexible circuit around a mandrel and covering with a polymer layer to form a tube.

The coils 152 formed on the cylindrical flexible circuit 130 can be radially disposed about or linearly disposed along a longitudinal axis 148' of the body 126 or cylindrical base. In this regard, each coil 152 is formed on separate discrete layers of the tubular body 126. The coils 152 can be axially displaced and rotationally positioned about the circumference of the tubular body 126. The coils 152 define a three coil assembly as described above that cooperate with the navigation system 10 such that 6 DOF tracking information can be determined.

In a configuration where three coils are utilized, two of the three coil assemblies can be positioned at an angle relative to the longitudinal axis 148 with the third coil assembly being positioned at an angle relative to the longitudinal axis 148 or parallel thereto. The three coils 152 can also each be positioned at an angle relative to each other. As shown in Figure 5, an exemplary angle of the three coils 152 relative to the longitudinal axis 148 can be 45 or 55 degrees, which also provides for optimal packaging and spacing of the coil assemblies circumferentially around support 190. It should be appreciated that while an angle of 45 or 55 degrees has been discussed, other angles could be utilized with coils 152 and instrument 100 as may be required.

In a configuration where tracking device 106 includes two coils 152, the two coils can similarly be positioned equidistant or 180 degrees spaced around an outer perimeter of exterior surface exterior surface 140. Additionally, they can be positioned at an angle relative to each other and at an angle relative to the longitudinal axis 148 of the tube assembly 110. In one exemplary configuration, the two coils 152 can be positioned at an angle of about 0 to 90 degrees, including about 45 degrees relative to longitudinal axis 148 of the tube assembly 110.

The twisted pairs 174 of traces 150A-C formed on the tubular body 126 can reduce electrical interference or cross-talk between each pair of adjacent lead traces 150A-C. Each pair of lead traces 150 can be connected to a single coil 152. Optionally for deformable instruments 100, the lead traces and tubular body 126 can also include a TEFLON^{®} coating or other appropriate lubricous or friction reducing coating on an outer surface thereof.

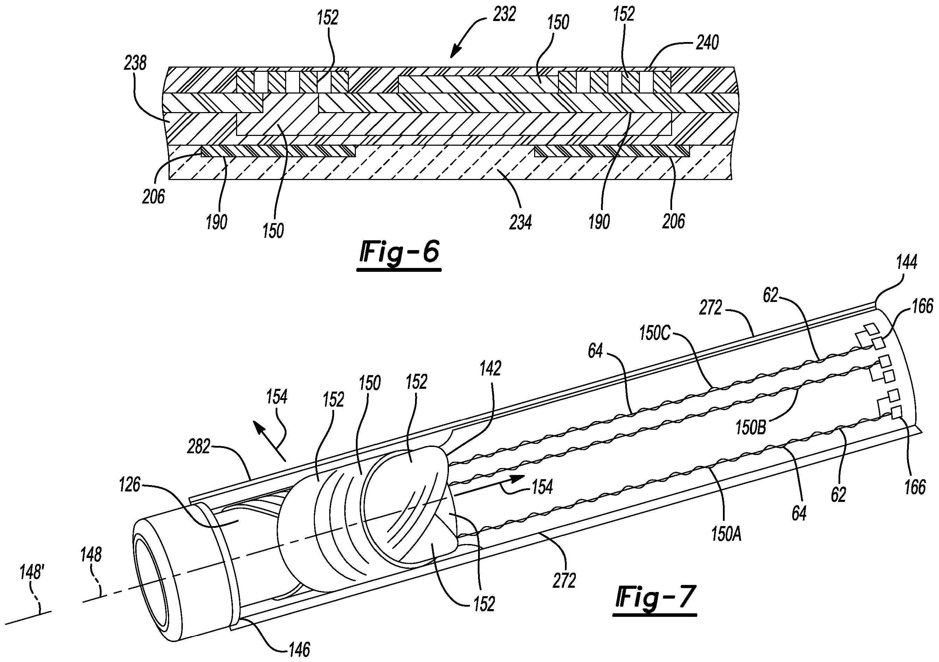

As shown in Figures 5 and 7, pairs of twisted traces 150A-C, can be helically defined around tubular body 126 from the coils 152 to the proximal end 144. The traces 150 can be defined around the exterior surface 140 of the tubular body 140 at an angle α relative to the longitudinal axis 148 of approximately 0 to 85 degrees, including about 30 degrees. Each revolution of the traces 150 around body can be spaced apart from each other by a distance d of approximately 2 to 45 mm, including about 5 mm. In one exemplary configuration, the range can include from about 15 - 45 mm. In this regard, the revolution spacing can be from about 2 mm. A further discussion of traces on a flexible circuit board can be found in U.S. Patent Application Serial No. 13/751,032, entitled "Flexible Circuit Sheet," filed on January 25, 2013.

Referring again to Figure 6, a cross-sectional view of the planar flexible circuit 232 is shown in a configuration utilizing a pressure sensitive adhesive 234 which can be used to couple the flexible circuit 232 to the instrument 100 in addition to the shrink-wrap tubing 272. In the event a tubular construction is desired, it could be similarly configured with two opposed sides. The tubular body 126 can be formed of various layers to form a laminate structure. These layers, for instance, can contain a KAPTON^{®} insulating film layer having the traces 150 deposited or etched thereon. Additional insulating layers 240 can be deposited over the traces 150. Optionally, the traces 150 can be formed on alternate layers so as to allow the coils 152 to be at least partially placed over one another. In this configuration, the substrate or base layer 142 can include a thickness of approximately 0.01 mm, the conductive traces 150 and coils 152 can include a thickness of approximately 0.04 mm, and the insulative layer 240 can include a thickness of approximately 0.02 mm. In the assembled configuration, the flexible printed circuit sheet can include an overall thickness of approximately 0.07 mm without adhesive and an overall thickness of 0.11 mm with adhesive 234.

Tubular body 126 can include a support layer 190 received on an exterior surface thereof to stiffen areas associated with the coils 152. This support layer 190 can be disposed at a distal end 146 of the tubular substrate 142. The support layer 190 can be included under the coils 152 in the form of a plurality of stiffened sections 206 configured to facilitate supporting a portion of the tracking sensor 106. The stiffened sections 206 provide a stable platform to resist deformation of the tracking devices 106 and, particularly, the deformation of the plurality of stiffened sections 206 configured to facilitate supporting a portion of the tracking sensor 106. The stiffened sections 206 provide a stable platform to resist deformation of the tracking devices 106 and, particularly, the deformation of the coils 152. In this configuration, the tracking device 106 can include three coils 152, as will be described herein.

As shown in Figure 7, in one exemplary configuration of the instrument 100, the tracking device 106 has three coils 152 formed by three traces 150 in an overlapping configuration. It is envisioned the coils could be formed in an interleaved configurations by the use of vias. The tracking device 106 can be coupled to the instrument using the shrink-wrapped polymer layer 272. The coils 152 which define navigation axis 154 have an overall axial length of approximately 1.5 mm to 2 mm, an overall diameter of approximately 0.3 to 0.5 mm, and a plurality of trace windings defined on one of the layers of the tubular body 126 along a cylindrical base to form the cylindrical configuration. As described above, the three coils 152 are each defined on radially disposed alternate layers. Each of these layers being a different distance from the longitudinal axis 148. Twisted pair traces 150A-C couple the coils 152 to the electrical coupling pads 166. The coupling pads 166 can interact for instance with a clip connector (not shown) or be a place for solder connections.

The tracking device 106 can be coupled to any medical device having an elongated member. In the case of a flat tracking device 106, the substrate can be wrapped around the elongated portion and coupled closed along a seam with adhesive or heat. In the case of a tracking device 106 formed of a closed tube, the substrate 128 can be slid over an elongated portion of an instrument 100. The substrate can be coupled to the medical device using adhesive or the heat shrink tube as described above. The tracking device 106 can then be electrically coupled to the navigation system 10 by use of an electrical connector. Optionally, the tracking device can be placed within a cavity defined within a body forming a medical device. In this regard, the device can also be placed within a polymer medical device. The tracking device can be placed within a mold and plastic injected into the mold, thus encapsulating the tracking device with a protective covering. As can be seen, the tracking of medical devices not normally associated with navigation systems is possible.

In use, the patient 34 can be positioned on an operating table or other appropriate structure and appropriate image data of a patient or navigation space can be obtained. The image data can be registered to the navigation space as is known in the art. The surgeon 50 can determine a shape of the instrument 100 to reach a target site and bend the instrument 100 to the determined shape where instrument 100 retains the bent shape, as discussed above. The surgical instrument 100 can then be guided to the target site with an icon representing the position of the distal tip of instrument 100 being superimposed on the image data. The icon can show the tracked relative position of the distal tip as instrument 100 is navigated to the target site. In addition, if during navigation of the shaped instrument 100 to the target site, the surgeon determines that the shaped configuration will need to be altered, the surgeon can bend and/or reshape the instrument 100 to a newly shaped configuration and proceed again as discussed above.

## Claims

1. A tracking device (106) for use with a medical instrument having a shaft and a medical device navigation system, the tracking device comprising:
an elongated closed tubular substrate (142) having an exterior surface and a longitudinal axis (148), wherein the elongated closed tubular substrate has a pair of open ends and is configured to be slid over the shaft along the longitudinal axis, in use; and
a first electrical trace (150) defining a first coil (152) circumferentially disposed on the elongated closed tubular substrate, having a first navigation axis (154);
further comprising:
a second electrical trace defining a second coil (152) circumferentially disposed on the elongated closed tubular substrate, having a second navigation axis (154); and
a third electrical trace defining a third coil (152) circumferentially disposed on the elongated closed tubular substrate, having a third navigation axis (154);
wherein the first coil, the second coil, and the third coil are each formed on separate discrete layers of the elongated closed tubular substrate, the first, second and third coils configured to interact with the navigation system;
wherein each of the first, second and third navigation axes are different axes.

2. The tracking device of claim 1 wherein the first, second and third navigation axes are not all parallel to each other.

3. The tracking device according to claim 1 or 2, wherein the first navigation axis is parallel to the longitudinal axis of the tubular substrate.

4. The tracking device according to any of claims 1 to 3, wherein the second navigation axis is non-parallel to the longitudinal axis of the tubular substrate.

5. The tracking device according to any one of claims 1 to 4, wherein the third navigation axis is non-parallel to the second navigation axis.

6. The tracking device according to any of claims 1 to 5, wherein the third navigation axis is perpendicular to the second navigation axis.

7. The tracking device according to any preceding claim, wherein the first, second and third coils are first, second and third distances, respectively, from a proximal end of the closed tubular substrate, and where the first, second and third distances are different.

8. The tracking device of claim 1, wherein the first, second and third navigation axes are all orthogonal to each other.

9. The tracking device (106) of any preceding claim, further comprising a medical instrument, said medical instrument comprising a surgical instrument (100) and a shaft, said shaft comprising:
an elongated body having a proximal end and a distal end;
the tracking device (106) positioned adjacent the distal end and adapted to cooperate with the navigation system to track the distal end of the body,
a handle coupled to the proximal end of the elongated body, the closed tubular substrate including at least a pair of lead traces disposed along the body from each coil to the handle.

10. The tracking device of claim 9, wherein the closed tubular substrate is a closed and continuous tube configured to be slid onto the elongated body.

11. A method of manufacturing a tracking device, comprising:
forming an elongated closed tubular substrate (142), having an exterior surface and a longitudinal axis (148), to have three individual trace-defined coils on separate discrete layers thereof, each trace-defined coil being circumferentially disposed around the elongated closed tubular substrate and having a navigation axis (154);
wherein each navigation axis (154) of each trace-defined coil is a different axis.

12. The method of claim 11, wherein said forming comprises disposing said trace-defined coils in separate discrete layers onto a planar flexible substrate (232) and subsequently winding the planar flexible substrate to form the elongated closed tubular substrate (142).

13. The method of claim 11, wherein said forming comprises disposing said trace-defined coils in separate discrete layers directly onto an elongated closed tubular substrate (128).

14. The method of any of claims 11 to 13, wherein each of the trace-defined coils (152) are formed to be have navigation axes (154) that are not parallel to one another.

15. The method of any of claims 11 to 14, wherein three pairs of lead traces are disposed on the tubular body, one connected to each trace-defined coil, said lead traces being configured to connect each coil to a surgical navigation instrument.

## Patentansprüche

1. Verfolgungsvorrichtung (106) zur Verwendung mit einem medizinischen Instrument, das einen Schaft und ein Navigationssystem für eine medizinische Vorrichtung aufweist, wobei die Verfolgungsvorrichtung Folgendes umfasst:
ein längliches geschlossenes röhrenförmiges Substrat (142), das eine Außenoberfläche und eine Längsachse (148) aufweist, wobei das längliche geschlossene röhrenförmige Substrat in Verwendung ein Paar offener Enden aufweist und konfiguriert ist, um entlang der Längsachse über den Schaft geschoben zu werden; und
eine erste elektrische Spur (150), die eine erste Spule (152) definiert, die in Umfangsrichtung auf dem länglichen geschlossenen röhrenförmigen Substrat angeordnet ist, die eine erste Navigationsachse (154) aufweist;
ferner Folgendes umfassend:
eine zweite elektrische Spur, die eine zweite Spule (152) definiert, die in Umfangsrichtung auf dem länglichen geschlossenen röhrenförmigen Substrat angeordnet ist, die eine zweite Navigationsachse (154) aufweist; und
eine dritte elektrische Spur, die eine dritte Spule (152) definiert, die in Umfangsrichtung auf dem länglichen geschlossenen röhrenförmigen Substrat angeordnet ist, die eine dritte Navigationsachse (154) aufweist;
wobei die erste Spule, die zweite Spule und die dritte Spule jeweils auf separaten diskreten Schichten des länglichen geschlossenen röhrenförmigen Substrats ausgebildet sind, wobei die erste, die zweite und die dritte Spule konfiguriert sind, um mit dem Navigationssystem zu interagieren;
wobei jede der ersten, zweiten und dritten Navigationsachsen unterschiedliche Achsen sind.

2. Verfolgungsvorrichtung nach Anspruch 1, wobei die erste, die zweite und die dritte Navigationsachse nicht alle parallel zueinander sind.

3. Verfolgungsvorrichtung nach Anspruch 1 oder 2, wobei die erste Navigationsachse parallel zu der Längsachse des röhrenförmigen Substrats ist.

4. Verfolgungsvorrichtung nach einem der Ansprüche 1 bis 3, wobei die zweite Navigationsachse nicht parallel zu der Längsachse des röhrenförmigen Substrats ist.

5. Verfolgungsvorrichtung nach einem der Ansprüche 1 bis 4, wobei die dritte Navigationsachse nicht parallel zu der zweiten Navigationsachse ist.

6. Verfolgungsvorrichtung nach einem der Ansprüche 1 bis 5, wobei die dritte Navigationsachse senkrecht zu der zweiten Navigationsachse ist.

7. Verfolgungsvorrichtung nach einem der vorhergehenden Ansprüche, wobei die erste, die zweite und die dritte Spule einen ersten, einen zweiten beziehungsweise einen dritten Abstand von einem proximalen Ende des geschlossenen röhrenförmigen Substrats aufweisen, und wobei der erste, der zweite und der dritte Abstand unterschiedlich sind.

8. Verfolgungsvorrichtung nach Anspruch 1, wobei die erste, die zweite und die dritte Navigationsachse alle orthogonal zueinander sind.

9. Verfolgungsvorrichtung (106) nach einem der vorhergehenden Ansprüche, die ferner ein medizinisches Instrument umfasst, wobei das medizinische Instrument ein chirurgisches Instrument (100) und einen Schaft umfasst, wobei der Schaft Folgendes umfasst:
einen länglichen Körper, der ein proximales Ende und ein distales Ende aufweist;
wobei die Verfolgungsvorrichtung (106) angrenzend an das distale Ende positioniert und angepasst ist, um mit dem Navigationssystem zusammenzuwirken, um das distale Ende des Körpers zu verfolgen,
einen Griff, der mit dem proximalen Ende des länglichen Körpers gekoppelt ist, wobei das geschlossene röhrenförmige Substrat wenigstens ein Paar von Leiterspuren einschließt, die entlang des Körpers von jeder Spule zu dem Griff angeordnet sind.

10. Verfolgungsvorrichtung nach Anspruch 9, wobei das geschlossene röhrenförmige Substrat ein geschlossenes und fortlaufendes Rohr ist, das konfiguriert ist, um auf den länglichen Körper geschoben zu werden.

11. Verfahren zum Herstellen einer Verfolgungsvorrichtung, das Folgendes umfasst:
Ausbilden eines länglichen geschlossenen röhrenförmigen Substrats (142), das eine Außenoberfläche und eine Längsachse (148) aufweist, um drei einzelne spurdefinierte Spulen auf separaten diskreten Schichten davon aufzuweisen, wobei jede spurdefinierte Spule in Umfangsrichtung um das längliche geschlossene röhrenförmige Substrat angeordnet ist und eine Navigationsachse (154) aufweist;
wobei jede Navigationsachse (154) jeder spurdefinierten Spule eine unterschiedliche Achse ist.

12. Verfahren nach Anspruch 11, wobei das Ausbilden das Anordnen der spurdefinierten Spulen in separaten diskreten Schichten auf einem ebenen flexiblen Substrat (232) und ein anschließendes Aufwickeln des ebenen flexiblen Substrats umfasst, um das längliche geschlossene röhrenförmige Substrat (142) auszubilden.

13. Verfahren nach Anspruch 11, wobei das Ausbilden das Anordnen der spurdefinierten Spulen in separaten diskreten Schichten direkt auf einem länglichen geschlossenen röhrenförmigen Substrat (128) umfasst.

14. Verfahren nach einem der Ansprüche 11 bis 13, wobei jede der spurdefinierten Spulen (152) ausgebildet ist, um Navigationsachsen (154) aufzuweisen, die nicht parallel zueinander sind.

15. Verfahren nach einem der Ansprüche 11 bis 14, wobei drei Paare von Leiterspuren auf dem röhrenförmigen Körper angeordnet sind, wobei eine mit jeder spurdefinierten Spule verbunden ist, wobei die Leiterspuren konfiguriert sind, um jede Spule mit einem chirurgischen Navigationsinstrument zu verbinden.

## Revendications

1. Dispositif de suivi (106) destiné à être utilisé avec un instrument médical ayant une gaine et un système de navigation de dispositif médical, le dispositif de suivi comprenant :
un substrat tubulaire fermé allongé (142) ayant une surface extérieure et un axe longitudinal (148), le substrat tubulaire fermé allongé ayant une paire d'extrémités ouvertes et étant conçu pour glisser sur la gaine le long de l'axe longitudinal, en cours d'utilisation ; et
un premier ruban électrique (150) définissant une première bobine (152) disposé circonférentiellement sur le substrat tubulaire fermé allongé, ayant un premier axe de navigation (154) ;
comprenant en outre :
un deuxième ruban électrique définissant une deuxième bobine (152) disposé circonférentiellement sur le substrat tubulaire fermé allongé, ayant un deuxième axe de navigation (154) ; et
un troisième ruban électrique définissant une troisième bobine (152) disposé circonférentiellement sur le substrat tubulaire fermé allongé, ayant un troisième axe de navigation (154) ;
la première bobine, la deuxième bobine et la troisième bobine étant chacune formées sur des couches distinctes séparées du substrat tubulaire fermé allongé, les première, deuxième et troisième bobines étant configurées pour interagir avec le système de navigation ;
chacun des premier, deuxième et troisième axes de navigation étant des axes différents.

2. Dispositif de suivi selon la revendication 1, dans lequel les premier, deuxième et troisième axes de navigation ne sont pas tous parallèles les uns aux autres.

3. Dispositif de suivi selon la revendication 1 ou 2, dans lequel le premier axe de navigation est parallèle à l'axe longitudinal du substrat tubulaire.

4. Dispositif de suivi selon l'une quelconque des revendications 1 à 3, dans lequel le deuxième axe de navigation est non parallèle à l'axe longitudinal du substrat tubulaire.

5. Dispositif de suivi selon l'une quelconque des revendications 1 à 4, dans lequel le troisième axe de navigation est non parallèle au deuxième axe de navigation.

6. Dispositif de suivi selon l'une quelconque des revendications 1 à 5, dans lequel le troisième axe de navigation est perpendiculaire au deuxième axe de navigation.

7. Dispositif de suivi selon l'une quelconque des revendications précédentes, dans lequel les première, deuxième et troisième bobines sont à des première, deuxième et troisième distances, respectivement, d'une extrémité proximale du substrat tubulaire fermé, et où les première, deuxième et troisième distances sont différentes.

8. Dispositif de suivi selon la revendication 1, dans lequel les premier, deuxième et troisième axes de navigation sont tous orthogonaux l'un par rapport à l'autre.

9. Dispositif de suivi (106) selon l'une quelconque des revendications précédentes, comprenant en outre un instrument médical, ledit instrument médical comprenant un instrument chirurgical (100) et une gaine, ladite gaine comprenant :
un corps allongé ayant une extrémité proximale et une extrémité distale ;
le dispositif de suivi (106) positionné de manière adjacente à l'extrémité distale et adapté pour coopérer avec le système de navigation pour suivre l'extrémité distale du corps,
une poignée accouplée à l'extrémité proximale du corps allongé, le substrat tubulaire fermé comportant au moins une paire de rubans conducteurs disposés le long du corps de chaque bobine à la poignée.

10. Dispositif de suivi selon la revendication 9, dans lequel le substrat tubulaire fermé est un tube fermé et continu conçu pour glisser sur le corps allongé.

11. Procédé de fabrication d'un dispositif de suivi, comprenant :
Le formage d'un substrat tubulaire fermé allongé (142), ayant une surface extérieure et un axe longitudinal (148), pour avoir trois bobines individuelles définies par un ruban sur des couches distinctes séparées de celui-ci, chaque bobine définie par un ruban étant disposée circonférentiellement autour du substrat tubulaire fermé allongé et ayant un axe de navigation (154) ;
dans lequel chaque axe de navigation (154) de chaque bobine définie par un ruban est un axe différent.

12. Procédé selon la revendication 11, dans lequel ledit formage comprend la disposition desdites bobines définies par un ruban en couches distinctes séparées sur un substrat flexible plan (232) et l'enroulement ultérieur du substrat flexible plan pour former le substrat tubulaire fermé allongé (142).

13. Procédé selon la revendication 11, dans lequel ledit formage comprend la disposition desdites bobines définies par un ruban en couches distinctes séparées directement sur un substrat tubulaire fermé allongé (128).

14. Procédé selon l'une quelconque des revendications 11 à 13, dans lequel chacune des bobines définies par un ruban (152) sont formées pour avoir des axes de navigation (154) qui ne sont pas parallèles les uns aux autres.

15. Procédé selon l'une quelconque des revendications 11 à 14, dans lequel trois paires de rubans conducteurs sont disposées sur le corps tubulaire, une reliée à chaque bobine définie par un ruban, lesdits rubans conducteurs étant conçus pour relier chaque bobine à un instrument de navigation chirurgical.
